# EUROPEAN PATENT APPLICATION

(11) **EP 2 163 549 A1**
(43) Date of publication of application: **17.03.2010**
(21) Application number: 09169613.8
(22) Date of filing: 07.09.2009
(51) Int. Cl.: C07D 307/60

(54) **Process for the Preparation of Tetronic Acid**

(30) Priority: 16.09.2008 JP 2008236613
(71) Applicant: DAICEL CHEMICAL INDUSTRIES, LTD., Osaka-shi, Osaka 530-0001 (JP)
(72) Inventor: Fujita, Kouhei, Himeji-shi Hyogo 671-1283 (JP); Ohno, Mitsuru, Himeji-shi Hyogo 671-1283 (JP)
(74) Representative: Grünecker, Kinkeldey, Stockmair & Schwanhäusser Anwaltssozietät

(57) **Abstract**

Tetronic acid of following Formula (2): 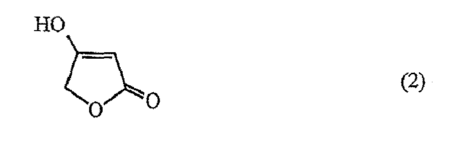 is industrially efficiently prepared through a reaction of 4-hydroxy- or 4-alkoxy-acetoacetic acid or an ester thereof of following Formula (1): 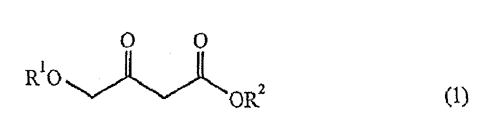 wherein R¹ and R² each independently represent a hydrogen atom or an alkyl group, by carrying out the reaction in the presence of an organic solvent and an acid but substantially in the absence of water. Product tetronic acid is useful as an intermediate for fine chemicals such as pharmaceuticals, agricultural chemicals, and semiconductor resist polymers.

## Description

### BACKGROUND OF THE INVENTION

### 1. Field of the Invention

The present invention relates to a process for the preparation of tetronic acid. Tetronic acid is useful as an intermediate for fine chemicals such as pharmaceuticals and agricultural chemicals and as an intermediate for semiconductor resist polymers.

### 2. Description of the Related Art

An exemplary known process for preparing tetronic acid is a process of preparing tetronic acid by reacting ethyl 4-tert-butoxyacetoacetate with an aqueous solution of hydrogen chloride(hydrochloric acid) and evaporating the aqueous solution of hydrogen chloride to dryness (Japanese Unexamined Patent Application Publication (JP-A) No. S58-971; and JP-A No. S58-972). However, the process is not advantageous in commercial-scale production, because tetronic acid is soluble in water; when water is used in the reaction as in this process, the resulting reaction mixture as an aqueous solution must be evaporated to dryness in order to obtain tetronic acid as crystals; and the evaporation of such a large amount of water requires extremely great energy.

JP-A No. S60-184073 discloses a process for preparing tetronic acid by chlorinating an ester of 4-chloroacetoacetic acid to 2,4-dichloroacetoacetic acid, subjecting the same to a heat treatment to give 3-chlorotetronic acid, dissolving resulting 3-chlorotetronic acid in water, carrying out a hydrogenation reaction, and evaporating the reaction mixture to dryness on a rotary evaporator. This process, however, is also not advantageous in commercial-scale production, because extremely great energy is required for the evaporation of a large amount of water, as in the above process.

Independently, JP-A No. H03-52881 discloses a process for preparing tetronic acid, in which water is not used in the reaction. According to this process, a lower alkyl ester of 4-halo-3-alkoxy-2-butenecarboxylic acid is subjected to cyclization to give an alkyl ester of tetronic acid, the alkyl ester of tetronic acid is hydrolyzed with an acid. More specifically the alkyl ester of tetronic acid is hydrolyzed with hydrogen chloride or hydrogen bromide in acetic acid, the solvent acetic acid is evaporated to dryness, tetronic acid is precipitated from methylene chloride, and collected through filtration. However, this process is also not advantageous in commercial-scale production, because it requires extremely great energy for the evaporation of acetic acid to dryness and uses a large amount of methylene chloride that significantly affects the environment.

Chem. Pharm. Bull., 1990, pages 99-102 discloses a process for preparing tetronic acid by refluxing 6-hydroxymethyl-2,2-dimethyl-1,3-dioxin-4-one in solvent toluene, evaporating the solvent to dryness, and carrying out recrystallization of tetronic acid from ethyl acetate. This process, however, is also not advantageous in commercial production, because it requires extremely great energy for the evaporation of the solvent to dryness and, in addition, the synthesis of material 6-hydroxymethyl-2,2-dimethyl-1,3-dioxin-4-one requires many steps.

### SUMMARY OF THE INVENTION

Accordingly, an object of the present invention is to provide a process for industrially efficiently preparing tetronic acid that is useful as an intermediate for fine chemicals such as pharmaceuticals and agricultural chemicals and as an intermediate for semiconductor resist polymers.

After intensive investigations to achieve the objects, the present inventors have found that a process for preparing tetronic acid by subjecting 4-hydroxy- or 4-alkoxy-acetoacetic acid or an ester thereof to cyclization using an organic solvent and an acid in the absence of water is very advantageous in commercial-scale production, because crystals of tetronic acid can be recovered without requiring evaporation of water to dryness, and the process thereby does not require extra energy for the evaporation or condensation of water. The present invention has been made based on these findings and further investigations.

Specifically, according to an embodiment of the present invention, there is provided a process for the preparation of tetronic acid represented by following Formula (2): 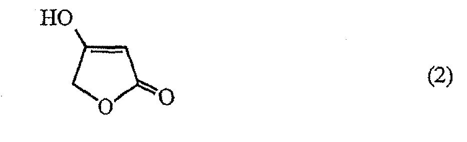 through a reaction of 4-hydroxy- or 4-alkoxy-acetoacetic acid, or an ester thereof, represented by following Formula (1) : 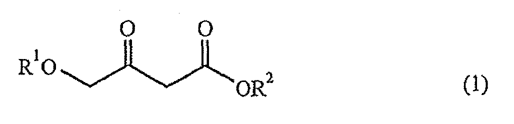 wherein R¹ and R² each independently represent a hydrogen atom or an alkyl group,
the process comprising the step of carrying out the reaction in the presence of an organic solvent and an acid but substantially in the absence of water.

The process preferably further includes the step of crystallizing tetronic acid from a poor solvent after the step of carrying out the reaction. Such crystallization is a more energy saving and more simple procedure than evaporation to dryness. The process more preferably further includes the step of reducing the amount of the organic solvent in the reaction system after the step of carrying out the reaction and before the step of crystallizing.

Acetic acid and hydrogen chloride gas are preferably used as the organic solvent and the acid, respectively.

The material 4-hydroxy- or 4-alkoxy-acetoacetic acid, or an ester thereof, represented by Formula (1) is preferably ethyl 4-tert-butoxyacetoacetate.

The process according to the present invention prepares tetronic acid from 4-hydroxy- or 4-alkoxy-acetoacetic acid or an ester thereof, using an organic solvent and an acid in the absence of water, thereby does not require evaporation of water to dryness for obtaining crystals of tetronic acid, and does not require much energy for the evaporation of a large amount of water. The process is therefore very advantageous in commercial-scale production.

In a preferred embodiment, the reaction is carried out in acetic acid as the organic solvent while blowing hydrogen chloride gas as the acid directly into the reaction system; the amount of acetic acid is reduced after the reaction; and a poor solvent is then added to carry out crystallization of tetronic acid. According to such embodiment, impurities can be easily separated from product tetronic acid into a filtrate; acetic acid used as the organic solvent enables an efficient reaction in a nonaqueous reaction system as in an aqueous reaction system and enables purification of product tetronic acid through crystallization; hydrogen chloride gas used as the acid can be blown directly into the reaction system and thereby enables efficient reaction. Thus, high-purity tetronic acid can be efficiently produced while minimizing consumed energy.

These and other objects, features, and advantages of the present invention will be understood more fully from the following description of the preferred embodiments. All numbers are herein assumed to be modified by the term "about."

### DESCRIPTION OF THE PREFERRED EMBODIMENTS

A process according to an embodiment of the present invention prepares tetronic acid represented by Formula (2) through a reaction of 4-hydroxy- or 4-alkoxy-acetoacetic acid, or an ester thereof, represented by Formula (1). This process includes the step of carrying out the reaction using an organic solvent and an acid in the absence of water.

### Organic Solvents

Exemplary organic solvents for use herein include ester solvents such as ethyl acetate; aromatic hydrocarbon solvents such as toluene; ether solvents such as 1,4-dioxane; and organic acid solvents such as acetic acid, formic acid, and propionic acid. Among them, organic acid solvents are preferred, of which acetic acid is especially preferred, because these organic acid solvents, especially acetic acid, allow the reaction to proceed efficiently and enable purification of resulting tetronic acid not through the evaporation of the organic solvent to dryness but through crystallization. Such crystallization is a more energy saving and more simple procedure than evaporation to dryness.

The amount of the organic solvent is not specifically limited, as long as reaction components can be dissolved or dispersed therein, and economical efficiency and other advantages are not impaired. Typically, the amount can be selected within a range of generally from about 1 to about 20 parts by weight, and preferably from about 2 to about 5 parts by weight, per 1 part by weight of 4-hydroxy- or 4-alkoxy-acetoacetic acid or an ester thereof. If the amount of the organic solvent is less than the above range, the yield and selectivity may deteriorate. In contrast, if the amount of the organic solvent is more than the above range, the concentration after the reaction may be done to a more extent, and the efficiency may thereby deteriorate.

### Acids

Exemplary acids for use herein include inorganic acids such as hydrogen chloride gas and sulfuric acid; and organic acids. An organic acid, if used as the solvent, can also be used as the acid. Among such acids, hydrogen chloride gas is preferably used herein, because it can be easily eliminated after the reaction.

The amount of the acid can be set as appropriate, and is, for example, generally from about 1 to about 50 parts by mole, preferably from about 1 to about 10 parts by mole, and especially preferably from about 1 to about 2 parts by mole, per 1 part by mole of 4-hydroxy- or 4-alkoxy-acetoacetic acid or an ester thereof. If the amount of the acid is lower than the above range, the reaction rate may often deteriorate, and this may impede efficient production of tetronic acid.

### 4-Hydroxy- or 4-alkoxy-acetoacetic acid, or ester of them

The starting material 4-hydroxy- or 4-alkoxy-acetoacetic acid or an ester thereof for use herein is represented by Formula (1). In Formula (1), R¹ and R² each independently represent a hydrogen atom or an alkyl group. Exemplary alkyl groups include linear alkyl groups containing from about 1 to about 20 carbon atoms, such as methyl, ethyl, propyl, butyl, pentyl, hexyl, octyl, nonyl, decyl, undecyl, and dodecyl groups, of which those containing from about 1 to about 10 carbon atoms are preferred, and those containing from about 1 to about 5 carbon atoms are more preferred; and branched-chain alkyl groups containing from about 3 to about 20 carbon atoms, such as isopropyl, isobutyl, sec-butyl, tert-butyl, and butyloctyl groups, of which those containing from about 3 to about 10 carbon atoms are preferred, and those containing from about 3 to about 5 carbon atoms are more preferred.

The alkyl groups may each have a variety of substituents. Exemplary substituents include halogen atoms, oxo groups, hydroxyl groups, substituted oxy groups (e.g., alkoxy groups, aryloxy groups, aralkyloxy groups, and acyloxy groups), carboxyl groups, substituted oxycarbonyl groups (e.g., alkoxycarbonyl groups, aryloxycarbonyl groups, and aralkyloxycarbonyl groups), substituted or unsubstituted carbamoyl groups, cyano groups, nitro groups, substituted or unsubstituted amino groups, sulfo groups, and heterocyclic groups. The hydroxyl groups and carboxyl groups may be protected by protecting groups commonly used in organic synthesis.

Among the compounds represented by Formula (1), ethyl 4-tert-butoxyacetoacetate is preferably used as the 4-hydroxy- or 4-alkoxy-acetoacetic acid, or an ester thereof, represented by Formula (1), since the reaction will proceed satisfactorily rapidly.

The 4-hydroxy- or 4-alkoxy-acetoacetic acid, or an ester thereof, represented by Formula (1) can be prepared by a know reaction or through the application of such a known reaction.

Of 4-hydroxy- or 4-alkoxy-acetoacetic acid and esters thereof represented by Formula (1), ethyl 4-tert-butoxyacetoacetate can be prepared, for example, by reacting ethyl 4-chloroacetoacetate with a metal tert-butoxide (t-BuOM), as in following Formula (3):

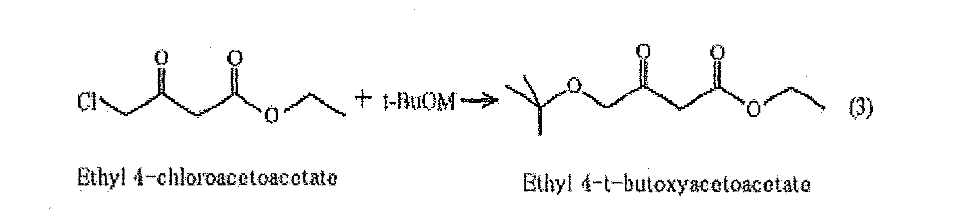

Examples of the metal element (M) in the metal tert-butoxide include sodium, potassium, and lithium. Among them, sodium is preferred as the metal element (M) for satisfactory reactivity, broad utility, and high economical efficiency.

The reaction temperature in the reaction between ethyl 4-chloroacetoacetate and the metal tert-butoxide can be suitably chosen according typically to the production rate of ethyl 4-tert-butoxyacetoacetate and the stability of the starting material ethyl 4-chloroacetoacetate and of the product ethyl 4-tert-butoxyacetoacetate. It can be chosen, for example, within a range of from about 0°C to about 100°C, preferably from about 0°C to about 50°C, and especially preferably from about 10°C to about 40°C.

The reaction is generally performed under normal atmospheric pressure or under a pressure (under a load) (e.g., at a pressure of from about 0.1 to about 50 MPa, and preferably from about 0.1 to about 10 MPa). The reaction may also be performed under reduced pressure for the sake of operational convenience.

The reaction may be performed in the presence of, or in the absence of, a solvent, but is generally performed in the presence of a solvent. Exemplary solvents include ether solvents such as diethyl ether, diisopropyl ether, dibutyl ether, tetrahydrofuran, dioxane, 1,2-dimethoxyethane, and cyclopentyl methyl ether (CPME); nitriles such as acetonitrile and benzonitrile; sulfoxide solvents such as dimethyl sulfoxide; sulfolanes such as sulfolane; ester solvents such as methyl acetate, ethyl acetate, and butyl acetate; lactones such as γ-butyrolactone; amide solvents such as dimethylformamide; alcohol solvents such as methanol, ethanol, propanol, isopropanol, n-butanol, s-butanol, and t-butanol; saturated or unsaturated hydrocarbon solvents such as pentane, hexane, heptane, octane, and petroleum ether; halogenated hydrocarbon solvents such as methylene chloride, chloroform, 1,2-dichloroethane, chlorobenzene, and bromobenzene; and high-boiling solvents such as poly(ethylene glycol)s and silicone oils. Among them, ether solvents such as tetrahydrofuran and cyclopentyl methyl ether (CPME) are preferably used. Each of different solvents can be used alone or in combination.

The amount of solvents is not especially limited, as long as reaction components can be dissolved or dispersed therein and the economical efficiency and other properties are not damaged. Typically, the amount of solvents can be chosen within a range of from about 1 to about 100000 parts by weight, and preferably from about 1 to about 10000 parts by weight, per 100 parts by weight of ethyl 4-chloroacetoacetate fed to the reaction system.

The reaction may be performed according to any of a batch system, a semi-batch system, and a continuous system. Where necessary, resulting ethyl 4-tert-butoxyacetoacetate may be purified according to a common separation/purification procedure such as separation through neutralization, extraction, distillation, rectification, molecular distillation, crystallization, recrystallization, and/or column chromatography.

### Reaction to Give Tetronic Acid

The reaction temperature in the reaction for the preparation of tetronic acid from 4-hydroxy- or 4-alkoxy-acetoacetic acid or an ester thereof can be appropriately chosen according typically to the stability of the starting material 4-hydroxy- or 4-alkoxy-acetoacetic acid or an ester thereof. The temperature is, for example, from about -10°C to about 100°C, preferably from about 5°C to about 50°C, and especially preferably from about 10°C to about 40°C. A reaction, if performed at a temperature below -10°C, proceeds at a significantly low rate, and this may impede efficient production of target tetronic acid. In contrast, a reaction, if performed at a temperature over 100°C, may tend to cause decomposition of the material, and this may lower the yield.

The reaction is generally performed under normal atmospheric pressure or under a pressure (under a load) (for example, at a pressure of from about 0.1 to about 50 MPa, and preferably from about 0.1 to about 10 MPa). The reaction may also be performed under reduced pressure for the sake of operational convenience.

The reaction may be performed according to any of a batch system, a semi-batch system, and a continuous system. Where necessary, resulting tetronic acid may be purified according to a common separation/purification procedure such as separation through neutralization, extraction, distillation, rectification, molecular distillation, crystallization, recrystallization, and/or column chromatography.

In a preferred embodiment of the present invention, the process further includes the step of crystallizing tetronic acid from a poor solvent after the step of carrying out the reaction. It is enough for the poor solvent to show a low solubility to tetronic acid. Examples of such poor solvents include toluene, ethyl acetate, cyclopentylmethyl ether (CPME), and n-hexane, of which toluene and ethyl acetate are preferably used for satisfactory yield, effective purification, and broad utility.

The amount of poor solvent can be suitably set according to the combination of the starting material 4-hydroxy- or 4-alkoxy-acetoacetic acid or an ester thereof and the poor solvent, and can be chosen within a range of, for example, generally from about 0.5 to about 10 parts by weight, and preferably from about 1 to about 5 parts by weight, per 1 part by weight of 4-hydroxy- or 4-alkoxy-acetoacetic acid or an ester thereof. A poor solvent, if its amount is excessively small, may not sufficiently contribute to efficient production of high-quality tetronic acid. Contrarily, a poor solvent, if its amount is excessively large, may cause economical disadvantage.

In another preferred embodiment, the process further includes the step of reducing the amount of the organic solvent in the system after the step of carrying out the reaction and before the step of crystallizing. Reduction of the amount of the organic solvent in the system enables efficient precipitation of crystals of tetronic acid.

The amount of the organic solvent in the system can be reduced, for example, by distilling off the organic solvent to concentrate the reaction mixture. The amount of the organic solvent after reduction typically through concentration can be suitably set according typically to the type of the starting material 4-hydroxy- or 4-alkoxy-acetoacetic acid or an ester thereof, and can be chosen within a range of, for example, generally from about 0.5 to about 10 parts by weight, and preferably from about 1 to about 2 parts by weight, per 1 part by weight of 4-hydroxy-or 4-alkoxy-acetoacetic acid or an ester thereof.

High-purity, high-quality tetronic acid can be prepared by subjecting crystals of tetronic acid precipitated by crystallization to a solid-liquid separation procedure such as filtration or centrifugal separation; subjecting the resulting solid to reprecipitation according to necessity; and drying.

### Examples

The present invention will be illustrated in further detail with reference to several examples below. It should be noted, however, these examples are never construed to limit the scope of the present invention.

NMR spectra were measured at 500 MHz (¹H-NMR) with a nuclear magnetic resonance system (trade name "BRUKER AM500" supplied by Bruker Corporation).

The amount of ethyl 4-tert-butoxyacetoacetate was determined using trans-stilbene as internal standard.

The purity of tetronic acid was determined quantitatively through high-performance liquid chromatography (HPLC) under the analytical conditions below using a commercial authentic sample.

### Conditions of HPLC Analysis

Column: trade name "TSK-GEL ODS-80Ts QA", 4.6 mm in diameter and 250 mm in length, supplied by Tosoh Corporation
Mobile phase: 0.2% aqueous phosphoric acid solution /acetonitrile = 95/5
Detection wavelength: UV (230 nm)
Detection temperature: 40°C
Flow rate: 1.0 ml/min.
Sample amount: 10 µl
Retention time: 5.7 min.
Sample concentration: 300 ppm

### Example 1

Sodium tert-butoxide (53.7 g, 0.559 mol) was suspended in tetrahydrofuran (429 g), to which a mixture of ethyl 4-chloroacetoacetate (40.0 g, 0.243 mol) and tetrahydrofuran (71.0 g) was added dropwise so that the temperature fell within a range of from 20°C to about 30°C. After the completion of dropwise addition, the mixture was stirred at room temperature (25°C) for 20 hours. After condensing tetrahydrofuran, a diluted hydrochloric acid (a mixture of 34.4 g of 35% hydrochloric acid and 200 g of water) was added dropwise under ice-cooling, followed by extraction with ethyl acetate and separation. The organic layer was washed with water, concentrated, and thereby yielded ethyl 4-tert-butoxyacetoacetate (yield: 43.1 g, yield by weight: 87.7%, purity: 76.6%).

¹H-NMR spectrum of ethyl 4-tert-butoxyacetoacetate (CDCl₃): δ 1.21 (s, 9H), 1.27 (t, 3H), 3.55 (s, 2H), 4.01 (s, 2H), 4.19 (q, 2H)

The above-prepared ethyl 4-tert-butoxyacetoacetate (40 g, 0.152 mol (converted based on purity)) was dissolved in acetic acid (210 g), followed by blowing hydrogen chloride gas (0.308 mol) thereinto at temperatures of from 20°C to 30°C. After the blowing, the mixture was stirred at room temperature (25°C) for 1 hour. The reaction mixture was placed in an evaporator, the pressure was reduced, residual hydrogen chloride gas was eliminated, low-boiling substances and acetic acid were then distilled out, and thereby the reaction mixture was concentrated to an amount of residual acetic acid of one-fifth (42 g) the charged amount. The residue was stirred to precipitate crystals, to which toluene (70 g) was added dropwise, followed by stirring at room temperature (25°C) for 2 hours. The crystals were collected by filtration and thereby yielded tetronic acid (yield: 11.5 g, yield by weight: 74.6%, purity: 98.1%).

### Example 2

Ethyl 4-tert-butoxyacetoacetate was prepared by the procedure of Example 1.

The above-prepared ethyl 4-tert-butoxyacetoacetate (10 g, 0.0399 mol (converted based on purity)) was dissolved in acetic acid (21.1 g), followed by blowing of hydrogen chloride gas (0.0867 mol) thereinto at temperatures of from 20°C to 30°C. After the blowing, the reaction mixture was stirred at room temperature (25°C) for 1 hour, placed in an evaporator, the pressure was reduced, residual hydrogen chloride gas was eliminated, low-boiling substances and acetic acid were then distilled out, and thereby the reaction mixture was concentrated to an amount of residual acetic acid of one half (10.55 g) the charged amount. The residue was stirred to precipitate crystals, to which ethyl acetate (9 g) was added dropwise, followed by stirring at room temperature (25°C) for 2 hours. The crystals were collected by filtration and thereby yielded tetronic acid (yield: 1.92 g, yield by weight: 47.5%, purity: 99.2%).

While the above description is of the preferred embodiments of the present invention, it should be appreciated that the invention may be modified, altered, or varied without deviating from the scope and fair meaning of the following claims.

## Claims

1. A process for the preparation of tetronic acid represented by following Formula (2): 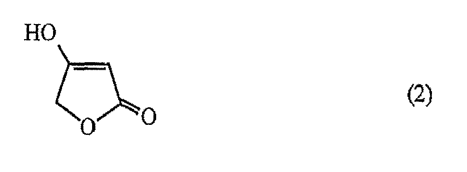 through a reaction of 4-hydroxy- or 4-alkoxy-acetoacetic acid, or an ester thereof, represented by following Formula (1): 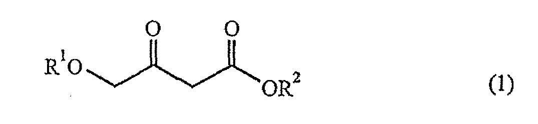 wherein R¹ and R² each independently represent a hydrogen atom or an alkyl group,
the process comprising the step of carrying out the reaction in the presence of an organic solvent and an acid but substantially in the absence of water.

2. The process according to claim 1, further comprising the step of crystallizing tetronic acid from a poor solvent after the step of carrying out the reaction.

3. The process according to claim 2, further comprising the step of reducing the amount of the organic solvent in the reaction system after the step of carrying out the reaction and before the step of crystallizing.

4. The process according to any one of claims 1 to 3, wherein acetic acid and hydrogen chloride gas are used as the organic solvent and the acid, respectively.

5. The process according to any one of claims 1 to 4, wherein ethyl 4-tert-butoxyacetoacetate is used as the 4-hydroxy- or 4-alkoxy-acetoacetic acid, or an ester thereof, represented by Formula (1).
